(19) Europäisches Patentamt
European Patent Office
Office européen des brevets

(11) Numéro de publication : **0 477 056 A1**

(12) **DEMANDE DE BREVET EUROPEEN**

(21) Numéro de dépôt : **91402336.1**

(22) Date de dépôt : **30.08.91**

(51) Int. Cl.$^5$ : **C12N 15/86,** C12N 9/12, C12N 15/38, A61K 39/255, A61K 39/17

(30) Priorité : **07.09.90 FR 9011146**

(43) Date de publication de la demande : **25.03.92 Bulletin 92/13**

(84) Etats contractants désignés : **AT BE CH DE DK ES GB GR IT LI LU NL SE**

(71) Demandeur : **RHôNE MERIEUX S.A.**
**17, rue Bourgelat**
**F-69002 Lyon (FR)**

(72) Inventeur : **Ross, Louis Joseph Norman**
**Spinney House, Rust Lane**
**Alconbury PE17 5DN, Huntingdon (GB)**
Inventeur : **Binns, Matthew McKinley**
**Appleby Cottage, Earith Road**
**Colne, Huntingdon PE17 3NJ (GB)**
Inventeur : **Rey-Senelonge, Arielle**
**35 allée de la Scarpe**
**F-69140 Rillieux la Pape (FR)**
Inventeur : **Riviere, Michel Emile Albert**
**11, chemin du Chancelier**
**F-69130 Ecully (FR)**

(74) Mandataire : **Bernasconi, Jean et al**
**CABINET LEMOINE ET BERNASCONI 13, Boulevard des Batignolles**
**F-75008 Paris (FR)**

(54) **Virus herpès recombinants, vaccins les comprenant, procédé de préparation et gènes US 3.**

(57) L'invention concerne la séquence de la région courte unique Us du virus de la maladie de Marek codant pour la protéine kinase. Elle concerne également les virus herpès recombinants dans lesquels un gène hétérologue a été inséré dans la région homologue du gène codant pour la protéine kinase, et particulièrement les virus de Marek recombinants exprimant un gène d'un agent pathogène aviaire, pouvant éventuellement être un gène d'un autre sérotype des virus de Marek. Elle concerne également un procédé de préparation de ces recombinants, ainsi que les vaccins obtenus.

EP 0 477 056 A1

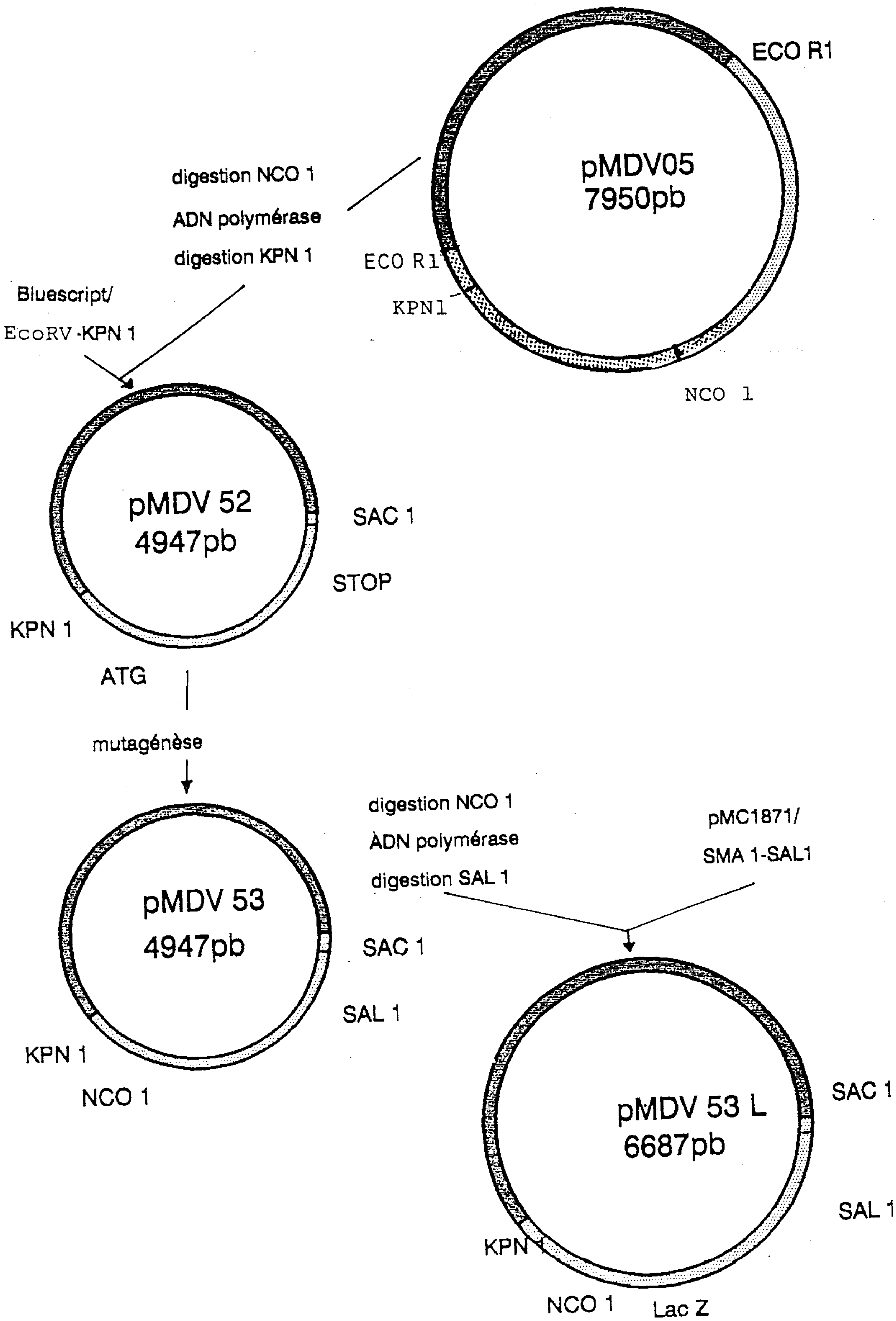

pMDV05
7950pb
ECO R1
ECO R1
KPN1
NCO 1
digestion NCO 1
ADN polymérase
digestion KPN 1
Bluescript/
EcoRV-KPN 1
pMDV 52
4947pb
SAC 1
STOP
KPN 1
ATG
mutagénèse
pMDV 53
4947pb
SAC 1
SAL 1
KPN 1
NCO 1
digestion NCO 1
ÀDN polymérase
digestion SAL 1
pMC1871/
SMA 1-SAL1
pMDV 53 L
6687pb
SAC 1
SAL 1
KPN 1
NCO 1
Lac Z

La présente invention concerne des virus herpès recombinants, notamment pour la réalisation de vaccins, leur procédé de préparation et les plasmides réalisés au cours de ce procédé. Elle concerne encore une partie du génome du virus de la maladie de Marek (MDV) qui est susceptible d'être utilisée pour la préparation de tels vaccins.

Différents types de virus ont été utilisés comme vecteurs d'expression de gènes étrangers, en particulier de gènes codant pour des protéines antigéniques, et ont démontré leur potentiel pour l'immunisation des animaux. Le virus de la vaccine a été largement utilisé pour la construction de virus recombinants. Les virus herpès ont été également employés : le virus herpès simplex (HSV) (M. Shih et al., Proc. Natl. Acad. Sci., USA, 1984, 81, 5867-5870), le virus de la varicelle (VZV) (R. Lowe et al., Proc. Natl. Acad. Sci., USA, 1987, 84, 3896-3900). Le gène étranger est inséré dans un fragment d'ADN génomique du virus herpès, correspondant à une région non essentielle pour la réplication virale, cloné dans un plasmide. Ce gène est transféré dans le génome viral par recombinaison homologue. Cette dernière étape est effectuée par cotransfection de l'ADN génomique herpès et du plasmide puisque cet ADN génomique est naturellement infectieux.

Différents gènes de virus herpès ont été identifiés comme non essentiels à la croissance virale, certains de ces gènes étant associés à la virulence.

– Le gène de la thymidine kinase du virus herpès simplex (D. Dubbs et al., Virology, 1965, 22, 493-502), du virus d'Aujeszky (G. Tatarov, Zentralbl. Vet. Med., 1968, 15B, 848853), du virus de la rhinotrachéite infectieuse bovine (S. Kit et al., Virology, 1983, 130, 381-389).
– Le gène gIII du virus d'Aujeszky (A. Robins et al., J. Virol., 1986, 59, 635-645).
– Le gène gX du virus d'Aujeszky (D. Thomsen et al., J. Virol., 1987, 61, 229-232).
– Le gène gI du virus d'Aujeszky (C. Mettenleiter et al., J. Virol., 1987, 61, 4030-4032).

Ces virus délétés gardent la capacité de produire une infection latente chez la souris.

Des études portant sur la région courte unique du génome du virus herpès simplex HSV-l ont été conduites (B. Megnier et al., Virology, 1988, 162, 251-254) et ont montré que des virus HSV-1 délétés dans la région courte ont subi une atténuation.

De leur côté, F.C. Purves et al. (Journal of Virology, 1987, vol. 61, n°9, 2896-2901) ont montré que la phase de lecture ouverte US3 du fragment court du génome du virus HSV-1 code pour une enzyme du virus, la protéine kinase, et n'est pas essentielle à la réplication dudit virus.

Des travaux ont été entrepris sur le virus de la maladie de Marek qui appartient à la sous-famille des gamma herpès virus. C'est un virus enveloppé possédant un ADN génomique linéaire double brin d'environ 175 kilobases. Son génome est composé d'un segment long (UL) et d'un segment court (Us) entourés par des séquences terminales inversées répétées.

Le virus de la maladie de Marek cause des paralysies et une maladie lymphoproliférative chez le poulet, généralement à l'âge de 2 à 5 mois. Cette maladie entraîne des pertes économiques très importantes (L. Payne, Biology of Marek's disease virus and the herpesvirus of turkeys, dans The Herpesvirus, vol. 1, p. 347-431, édité par B. Roizman, Plenum Press).

Les souches du virus de la maladie de Marek ont été classées en trois sérotypes :

– le sérotype 1 comprend les souches pathogènes et les souches atténuées qui en dérivent,
– le sérotype 2 comprend les souches naturellement atténuées,
– le sérotype 3 comprend le virus de l'herpès du dindon (HVT) et ses variants.

Par la suite, le terme virus de la maladie de Marek atténué désignera à la fois les sérotypes 1, 2 et 3.

Les virus de la maladie de Marek (MDV) et de l'herpès du dindon (HVT) possèdent des organisations génomiques analogues (A. Buckmaster et al., J. Gen. Virol. 1988, 69, 2033-2042) et de nombreuses homologies de séquence tout au long de leur génome (C. Gibbs et al., Proc. Acad. Natl. Sci USA 1984, 81, 3365-3369).

Les poussins sont vaccinés à l'âge d'un jour et sont protégés ensuite contre la maladie de Marek pendant leur vie entière. Pendant de nombreuses années, la vaccination à l'aide du virus herpès du dindon vivant (HVT) a été très efficace pour contrôler la maladie.

Néanmoins, l'émergence de nouvelles souches virales hautement virulentes a amené l'utilisation de souches de virus de la maladie de Marek atténuées d'un autre sérotype, pour vacciner et augmenter ainsi le niveau de protection, soit par exemple la souche CVI 988, MDV sérotype 1 atténué par passages sur cellules (B. Rispens et al., Avian Dis. 1972, 16, 1108-125), soit par exemple l'association des souches HVT MDV sérotype 3 et SB1, MDV sérotype 2 (K. Schat et al., J. Natl. Cancer Inst., 1987, 60, 1075-1082).

Le génome du virus de Marek possède certaines similarités avec le génome des alphavirus, herpès simplex (HSV) et varicelle (VZV). La plupart des gènes localisés dans la longue région UL du génome sont approximativement colinéaires entre les virus herpès simplex, varicelle (D. Mc Geoch, J. Gen. Virol., 1988, 69, 1531-1574) et virus de la maladie de Marek (A. Buckmaster, 1988). Ainsi, dans la demande de brevet internationale WO 90/02802, il a été proposé l'insertion de gènes dans cette région UL de HVT et de MDV.

Par contre, la localisation des gènes dans le segment Us montre une plus grande divergence entre virus

herpès. Aussi parmi les douze phases de lecture identifiées pour le virus herpès simplex, seulement quatre présentent une homologie avec le virus de la varicelle (Mc Geoch, 1988, déjà cité).

De fait, l'art antérieur ne suggère pas qu'il y ait un intérêt à procéder à une étude d'homologie des cadres de lecture ouverts de la région courte unique du génome du virus de la maladie de Marek avec des gènes de HSV-1.

La présente invention divulgue pour la première fois la séquence codant pour la protéine kinase du génome du virus de la maladie de Marek (MDV) et a permis d'établir, de façon surprenante, que ce gène pouvait être délété sans bloquer la réplication virale et permettre l'insertion de séquences hétérologues ouvrant la voie au développement d'une série de vecteurs d'expression viraux.

Un tel virus de la maladie de Marek recombinant et atténué constitue un candidat de choix pour le développement d'un vecteur viral exprimant des gènes étrangers destinés à la vaccination polyvalente des volailles puisqu'il présente l'avantage de pouvoir être utilisé pour ses propriétés vaccinales propres et comme vaccin contre d'autres maladies virales, bactériennes ou parasitaires, comme par exemple la bronchite infectieuse aviaire, la maladie de Newcastle, la peste aviaire, le syndrome chute de ponte, la maladie de Gumboro, l'anémie aviaire, la coccidiose, la variole aviaire, la laryngotrachéite infectieuse, la colibacillose, la pasteurellose, l'hémophilose.

## RESUME DE L'INVENTION

L'invention a pour objet de fournir des virus herpès recombinants, y compris un virus de la maladie de Marek atténué (sérotypes 1, 2 ou 3) recombinant utilisable comme vaccin, la méthode pour construire un tel virus recombinant ainsi qu'un virus vecteur permettant la vaccination multivalente contre des maladies aviaires virales, bactériennes ou parasitaires.

L'invention a pour objet la séquence nucléotidique et ses variantes correspondant au gène US3 homologue du gène de la protéine kinase du virus herpès simplex et aux régions environnantes. Par variantes de la séquence nucléotidique, on entend, comme il est d'usage, toute séquence équivalente, telle qu'obtenue par exemple par dégénérescence du code, modifications mineures, mutations ou correspondant à des variants viraux.

L'invention a pour objet un virus recombinant choisi parmi les virus herpès, y compris les virus de la maladie d'Aujesky, de la rhinotrachéite infectieuse des bovins, de la rhinopneumonie équine, de la rhinotrachéite féline, de l'herpès canin.

L'invention a également pour objet un virus de la maladie de Marek recombinant comprenant un ou plusieurs gènes hétérologues insérés dans la région de son génome correspondant au gène US3 de manière à pouvoir être exprimés.

Par "gène hétérologue", on entend notamment un gène codant pour une protéine ou une glycoprotéine immunogène d'un agent pathogène viral, bactérien ou parasitaire, notamment une agent associé à une pathologie aviaire. Cela concerne également la construction de virus hybrides, par exemple en introduisant, dans le génome d'un virus herpès du dindon, des gènes codant pour des immunogènes d'un virus de la maladie de Marek du sérotype 1 et/ou du sérotype 2.

On entend également par "gène hétérologue" un gène codant pour un peptide ou une protéine, par exemple hormone, facteur de croissance, immunomodulateur.

Le gène hétérologue est exprimé de préférence sous le contrôle des séquences régulatrices de la transcription du gène US3. On peut cependant faire en sorte que cette expression soit contrôlée par une séquence promotrice provenant d'un autre gène du virus considéré, par exemple le promoteur du gène TK, du gène gA, du gène gB, ou d'un autre virus herpès, par exemple le promoteur du gène gI du virus de la rhinotrachéite infectieuse bovine ou du gène II ou du gène III du virus d'Aujeszky.

De préférence, on substitue les codons d'initiation et de terminaison du gène US3 par ceux du gène à exprimer.

## DESCRIPTION DES FIGURES.

La figure 1 représente la construction du plasmide pMDV 53L qui contient le gène LacZ inséré à la place du gène US3.

La figure 2 représente la construction du plasmide pMDV 53CL qui contient le gène LacZ sous le controle du promoteur iE du cytomégalovirus humain à la place du gène US3.

La figure 3 représente la plasmide pMDV 53F qui contient le gène de la protéine fusion du virus de la maladie de Newcastle à la place du gène US3.

DESCRIPTION DE L'INVENTION : MATERIELS ET METHODES.

Souche virale

La souche sérotype 1 RB1B du virus de la maladie de Marek (MDV) a été utilisée (Schat K.A. et al, 1982, Avian Pathol. 11, 593-605).

Les méthodes de culture du virus et d'extraction d'ADN viral de haut poids moléculaire ont été décrites (C. Lee et al., 1980 J. Gen. Virol. 51, 235-253 ; N. Ross et al. 1989, 70, 1789-1804).

Culture cellulaire

Les fibroblastes d'embryon de poulet (CEP) ont été cultivés en milieu 199 F10, supplémenté en pénicilline, streptomycine, fungizone et sérum de veau foetal, comme décrit (N. Ross 1975, J. Gen. Virol. 28, 37-47).

Clonage de l'ADN viral

De manière générale, les techniques utilisées pour la construction des plasmides recombinants sont celles décrites par T. Maniatis et al. (T. Maniatis et al, 1982, Molecular cloning : a laboratory manual, Cold Spring Harbor Laboratory, Cold Spring Harbor, NY).

Pour toutes les étapes de clonage ou de sous-clonage, le vecteur linéarisé par les enzymes de restriction appropriées est déphosphorylé avant ligation. La purification des fragments d'ADN à partir d'un gel d'agarose est faite selon la technique décrite par le fabricant : "Geneclean" (Bio 101, San Diego, Californie, USA).

Séquençage.

Les fragments clonés sont séquencés selon la technique classique décrite par Sanger (G. Sanger, S.Nicklen, A. Coulson, 1977, Proc. Natl. Acad., USA 74, 5463-5467). Les séquences après traduction ont été comparées aux séquence publiées de virus herpès simplex et varicelle (Mc Geogh, 1985, J. Mol. Biol. 181, 1, 13 ; K. Davison et al. 1986, J. Gen. Virol. 67, 1759-1816).

Mutagénèse dirigée.

Les fragments d'ADN, sous-clonés dans le vecteur Blue Script (Stratagene, La Jolla, Californie, USA) sont mutagénéisés après séparation des ADNs simple brin à l'aide du phage helper R408 (Stratagene, La Jolla, Californie, USA) (M. Russel, S. Kidd, M. Kelley, 1986, Gene 45, 333-338).

La procédure de mutagénèse et de sélection des mutants en utilisant la souche CJ 236 dut-, ung- d'E. Coli (In Vitrogen, San Diego, Californie, USA) est décrite par T. Kunkel, (T. Kunkel 1985, Proc. Natl. Acad. Sci., 82, 488-492 et T. Kunkel et al., 1987, Methods of enzymology 154, 367-382, Acad. Press).

Recombinaison in vivo.

Les virus recombinants sont obtenus selon les techniques classiques de transfection des cellules sensibles, telles que la méthode au phosphate de calcium ou celle utilisant le réactif Lipofectine décrite par le fabricant BRL (P.L. Felgner et al., 1987, Proc. Natl. Acad. Sci., USA, 84, 7413). Pour cela, les fibroblastes d'embryons de poulet, cultivés jusqu'à confluence, sont cotransfectés avec de l'ADN génomique et le plasmide porteur du fragment d'ADN à insérer, flanqué en 5′ et 3′ des séquences du génome qui permettent la recombinaison.

Les virus recombinants peuvent ensuite être criblés par hybridation avec une sonde appropriée ou par la coloration des plages. Lorsqu'un gène marqueur, le gène lacZ de la β-galactosidase, est inséré dans le génome du virus de la maladie de Marek, l'expression de ce gène peut être suivie en additionnant, à la couche cellulaire, une surcouche d'agarose renfermant le substrat chromogène pour la β-galactosidase, e.g. Xgal (5-bromo-4-chloro-3-indolyl, B.D galactopyranoside).

**Exemple 1 : Isolement d'un fragment EcoRI de 5,25 kilobases.**

L'ADN génomique viral a été digéré par l'enzyme de restriction EcoRI et les fragments clonés dans le vecteur pUC 13 (Pharmacia) (Yannisch, Perron et al., 1985, Gene 33, 103-119).

Parmi les fragments clonés, un fragment de 5,25 kilobases, localisé au niveau du petit fragment Us, a été

plus particulièrement analysé par séquençage (pMDV 05 ; séquence ID n° 1).

La séquence renferme six cadres de lecture ouverte (ORF). Les séquences traduites de 4 de ces ORF présentent une homologie avec des protéines de virus HSV de type 1, localisées dans le fragment Us. En particulier le gène US3 du virus de la maladie de Marek présente une homologie avec le gène de la protéine kinase du virus herpès simplex.

De manière surprenante, l'étude de cette région a montré que le gène US3 pouvait être délété sans bloquer la réplication virale.

**Exemple 2 : Construction d'un plasmide pMDV 53L pour lequel le gène US3 a été remplacé par le gène lacZ (figure 1).**

Le fragment EcoRI de 5,25 kilobases issu du clone pMDV 05 a été digéré par l'enzyme NcoI et les extrémités ainsi générées réparées par l'ADN polymérase fragment Klenow. Il a ensuite été digéré par l'enzyme KpnI, et le fragment de 1989 paires de bases ainsi libéré a été cloné dans le vecteur Blue Script linéarisé par les enzymes EcoRV et KpnI pour donner le plasmide pMDV 52 de 4947 paires de bases.

Les sites NcoI et SalI ont été introduits respectivement aux extrémités 5′ et 3′ du fragment cloné par mutagénèse dirigée en utilisant les oligonucléotides désignés par Seq ID n° 2 et 3, dans la liste des séquences annexées, ce qui génère le plasmide pMDV 53. Le gène lacZ a été purifié à partir du plasmide pMC 1871 (Pharmacia LKB, Uppsala, Suède) (S.K. Shamira et al. 1983, Gene 25, 71-82) par digestion par les enzymes SmaI et SalI.

Il a ensuite été inséré dans le plasmide pMDV 53, digéré partiellement par l'enzyme NcoI, traité par l'ADN polymérase fragment Klenow, et digéré ensuite par l'enzyme SalI, ce qui génère le plasmide pMDV 53L d'environ 6687 paires de bases.

**Exemple 3 : Préparation d'un virus de la maladie de Marek renfermant le gène lacZ.**

Les fibroblastes d'embryon de poulet ont été cotransfectés avec l'ADN génomique total du virus et l'ADN du plasmide pMDV 53L linéarisé (10 à 50 μg). Les cultures ont été suivies pendant 4 à 6 jours jusqu'à l'apparition de foyers infectieux. Alternativement les cellules sont trypsinées après 72 h, puis réinoculées (1:1 ou 1:2) en un passage secondaire, et cela jusqu'à l'obtention de plages de lyse.

Le milieu est alors remplacé par du milieu neuf renfermant 1 % d'agarose et 0,5 % d'Xgal.

Les foyers dus aux virus recombinants se distinguent par leur coloration bleue.

Les virus peuvent alors être purifiés par plaque-purification et, après inoculation à des cellules saines, donnent des figures d'effet cytopathogène colorées en bleu en présence d'Xgal.

**Exemple 4 : Construction du plasmide pMDV 53 CL qui renferme le gène lacZ sous contrôle du promoteur immédiat précoce du cytomégalovirus humain (CMV) (figure 2).**

Le gène lacZ de la β-galactosidase a été placé sous le contrôle du promoteur immédiat précoce du cytomégalovirus humain (IECMV) dans le vecteur pCMV-LacZ.

Le fragment d'environ 4500 paires de bases renfermant l'ensemble, le promoteur IE du cytomégalovirus et le gène lacZ, a été digéré par l'enzyme EcoRI, et les extrémités remplies par l'ADN polymérase fragment Klenow. Il a ensuite été digéré par l'enzyme SalI.

Le fragment ainsi libéré a été cloné dans le vecteur pMDV 53 digéré partiellement, par l'enzyme NcoI, traité par l'ADN polymérase fragment Klenow, et digéré ensuite par l'enzyme SalI. Ce plasmide d'environ 8200 paires de bases construit est dénommé pMDV 53 CL.

**Exemple 5 : Construction d'un virus Marek recombinant pour lequel le gène lacZ a été introduit sous le contrôle du promoteur immédiat précoce du cytomégalovirus, à la place du gène US3.**

Les CEP ont été cotransfectées avec de l'ADN génomique du virus et de 10 à 50 μg d'ADN du plasmide pMDV 53 CL linéarisé.

On peut ainsi obtenir des virus recombinants qui se distinguent par l'apparition de foyers infectieux colorés en bleu en présence du substrat chromogène Xgal. Ces virus ont été purifiés selon la technique de purification de plages et ont permis d'infecter des fibroblastes d'embryon de poulet secondaires.

Des foyers infectieux colorés en bleu peuvent être obtenus en présence d'Xgal, ce qui montre que le gène lacZ est inséré à la place du gène US3.

**Exemple 6 : Construction du plasmide pMDV 53F qui renferme le gène de la protéine fusion du virus de la maladie de Newcastle à la place du gène US3 (figure 3).**

Le gène fusion (J. Taylor at al., 1990, J. Virol. 64, 1441-1450) a été introduit sous forme d'un fragment à extrémités franches dans le vecteur Blue Script au site SmaI pour donner le plasmide pNF1 de 5300 paires de bases.

Des sites NcoI et SalI ont été introduits par mutagénèse dirigée au niveau des codons ATG, et Stop, du gène fusion, grâce aux oligonucléotides répertoriés SEQ ID n° 4 et SEQ ID n° 5 respectivement dans la liste des séquences annexées.

Le fragment de 1682 paires de bases NcoI/SalI provenant de plasmide pNF2, a été inséré dans le vecteur pMDV 53 digéré partiellement par l'enzyme NcoI et digéré par l'enzyme SalI pour donner le plasmide pMDV 53F de 5369 paires de bases.

**Exemple 7 : Construction d'un virus de la maladie de Marek renfermant le gène de la protéine fusion du virus de la maladie de Newcastle.**

Les fibroblastes d'embryon de poulet ont été cotransfectés avec l'ADN génomique total du virus et de 10 à 50 μg d'ADN linéarisé du plasmide pMDV 53F. Les cultures ont été suivies jusqu'à apparition de foyers infectieux.

Les virus recombinants ont ensuite été criblés par hybridation avec une sonde renfermant le gène fusion.

Des procédures similaires sont utilisées pour la construction de virus herpès recombinants non aviaires, par insertion d'un gène hétérologue dans la région Us et en particulier dans le gène homologue au gène US3 du virus de Marek.

L'invention concerne également les vaccins, vivants ou non, constitués par ou contenant les virus recombinants construits selon l'invention, ou contenant des immunogènes exprimés par ces virus.

ANNEXE 1

LISTE DES SEQUENCES

SEQ ID n° 1

Longueur de la séquence : 5 255 paires de bases

Type de molécule séquencée : ADN génomique

Origine de la molécule : virus de la maladie de Marek, souche RB1 B

Source expérimentale : plasmide pMDV 05

Caractérisques :

de 1 à 324 paires de bases : région non codante

de 325 à 1 135 paires de bases : gène US1. Le gène est codé par le brin d'ADN complémentaire à la séquence indiquée et est transcrit de droite à gauche (SEQ ID n° 1.B)

de 623 à 1 214 paires de bases : gène US2
de 1 215 à 1 245 paires de bases : région non codante
de 1 246 à 2 451 paires de bases : gène US3
de 2 452 à 2 563 paires de bases : région non codante
de 2 564 à 3 004 paires de bases : gène US4
de 3 005 à 3 190 paires de bases : région non codante
de 3 191 à 4 384 paires de bases : gène US5
de 4 385 à 4 494 paires de bases : région non codante
de 4 495 à 5 253 paires de bases : gène US6

```
CAAAAATTTACATTAGTAATCTTTCTCGGTGGCTTACCAAATCGTCCTCTTGGTATATCCATATCATCGAAC    72

ATTGTAGCATTGACTCTGCTCATCGTTGTCTTTCAAATGCGCTCGATTGTTGAATCTCTCCTGATGTTAGAA   144

GTATATGGAAGATAGCCTGGATACATAAGTGATCTAGAAGGGTTTGTTATTGCAGTAATATACAAATTATAC   216

GTGACACTATAGCGACGGTTGTAGCGATGCACCTAATCGTAATGTGTATACGCCCCATCATGTAATTATATC   288

TAATTGGTAGCAAGTAGGTCTGTCGAATAACAGCTAATGACTACCGGCTCTACATTTTTTCTGTATTCGTGA   360

CTTTCCTGTCGCAGTGTAACGAACCGGAATTGCAATCGCATCTCTATCTTCTTTCTTGCAACATTTTCCACA   432

ACAGAATAATCTGCCGGGTGTACTACTCATTTGAGGTGGTTCGATTTCCGGAGGTTTTAGAGGATTGGGTGG   504

GGACCCGAGGATTTTGTATACACATACCATATCACTGTCGCAAAAATGCGCTCTATCTTCTGGGGTGTCGAA   576

CTTCGGTTCCCATGTAGATGTCAAGAGAGTTTGAATATTGTCGGGA ATG GCC CAC GGC ATA CCG     640
                                               Met Ala His Gly Ile Pro       6

GAC CAG GTC CCA GAC ACT TTG ATT GCA AGT AAC CTT TTT GGC AAA GGA ATA CAT     694
Asp Gln Val Pro Asp Thr Leu Ile Ala Ser Asn Leu Phe Gly Lys Gly Ile His      24

TCG AGC GCA ATG CGA CAT ATA TCT GCC GCC CCA ACT ATC CAC AAG CTA TGT GGA     748
Ser Ser Ala Met Arg His Ile Ser Ala Ala Pro Thr Ile His Lys Leu Cys Gly      42

GCA TTA CCA GAA ACT TCA GAT TCC AAC ATC AAA TAT CCA GAT AGA ACA TCC TGC     802
Ala Leu Pro Glu Thr Ser Asp Ser Asn Ile Lys Tyr Pro Asp Arg Thr Ser Cys      60

CAT TCT GTG GAA CAT CCT GCA ACA TCT TCA AAT AGC CGC ACT ATA AAC GAA TCC     856
His Ser Val Glu His Pro Ala Thr Ser Ser Asn Ser Arg Thr Ile Asn Glu Ser      78

CTA GTT CCG GCC AAT CCG GTA CCA CGA ACT CCA GTT CCA TCT GGT GGC TTT GTC     910
Leu Val Pro Ala Asn Pro Val Pro Arg Thr Pro Val Pro Ser Gly Gly Phe Val      96

CTT ACT ATC GGT CGA TGT TGC CGA GGA AGA ATT AAC ATG GGT TTG GCA AAA CGG     964
Leu Thr Ile Gly Arg Cys Cys Arg Gly Arg Ile Asn Met Gly Leu Ala Lys Arg     114

AAT AGG TCT GCA GCT CTG ACG ATT ATG GGC ACA CCC ACA TCA TCC TGT ATT TGT    1018
Asn Arg Ser Ala Ala Leu Thr Ile Met Gly Thr Pro Thr Ser Ser Cys Ile Cys     132

TCC ATA CAT TGC TTT ATA AGG AAT ATC CAT AAA GTA GAT GCA GCA TCT CTA GAT    1072
Ser Ile His Cys Phe Ile Arg Asn Ile His Lys Val Asp Ala Ala Ser Leu Asp     150

CTT CCT GGC AAT CGA TCG CAT TCA TCT AGA AGT GTG ACT ATA GTT ATC ATG GAC    1126
Leu Pro Gly Asn Arg Ser His Ser Ser Arg Ser Val Thr Ile Val Ile Met Asp     168

ACA CCC ATC TTC ACT CCA CCA ATA ATC TTT TTT ATT GTT AAT AAC TGG GCC GGT    1180
Thr Pro Ile Phe Thr Pro Pro Ile Ile Phe Phe Ile Val Asn Asn Trp Ala Gly     186

CTG ATC TCC AAA TCT TAT ACC TCT GGT AGA ATA  TGAAACAGGGTTAAAACTAGGTAATAG 1240
Leu Ile Ser Lys Ser Tyr Thr Ser Gly Arg Ile                                 197

ACTGGATG TCT TCG AGT CCG GAG GCA GAA ACG ATG GAA TGC GGC ATT TCT TCG TCG 1296
     Met Ser Ser Ser Pro Glu Ala Glu Thr Met Glu Cys Gly Ile Ser Ser Ser    214

AAA GTA CAC GAC TCT AAA ACT AAT ACT ACC TAC GGA ATT ATA CAT AAC AGC ATC    1350
Lys Val His Asp Ser Lys Thr Asn Thr Thr Tyr Gly Ile Ile His Asn Ser Ile     232

AAT GGT ACG GAT ACG ACG TTG TTT GAT ACT TTT CCC GAC AGT ACC GAT AAC GCG    1404
Asn Gly Thr Asp Thr Thr Leu Phe Asp Thr Phe Pro Asp Ser Thr Asp Asn Ala     250

GAA GTG ACG GGG GAT GTG GAC GAT GTG AAG ACT GAG AGC TCT CCC GAG TCC CAA    1458
Glu Val Thr Gly Asp Val Asp Asp Val Lys Thr Glu Ser Ser Pro Glu Ser Gln     268
```

```
TCT GAA GAT TTG TCA CCT TTT GGG AAC GAT GGA AAT GAA TCC CCC GAA ACG GTG  1512
Ser Glu Asp Leu Ser Pro Phe Gly Asn Asp Gly Asn Glu Ser Pro Glu Thr Val   286

ACG GAC ATT GAT GCA GTT TCA GCT GTG CGA ATG CAG TAT AAC AAT GTT TCA TCG  1566
Thr Asp Ile Asp Ala Val Ser Ala Val Arg Met Gln Tyr Asn Asn Val Ser Ser   304

TTA TCG CCC GGA TCT GAA GGG TAT ATC TAT GTT TGT ACA AAG CGT GGG GAT AAT  1620
Leu Ser Pro Gly Ser Glu Gly Tyr Ile Tyr Val Cys Thr Lys Arg Gly Asp Asn   322

ACC AAG AGA AAA GTC ATT GTG AAA GCT GTG ACT GGT GAC AAA ACC CTT GGG AGT  1674
Thr Lys Arg Lys Val Ile Val Lys Ala Val Thr Gly Asp Lys Thr Leu Gly Ser   340

GAA ATT GAT ATA TTA AAA AAA ATG TCT CAC CGC TCC ATA ATT AGA TTA GTT CAT  1728
Glu Ile Asp Ile Leu Lys Lys Met Ser His Arg Ser Ile Ile Arg Leu Val His   358

GCT TAT AGA TGG AAA TCG ACA GTT TGT ATG GTA ATG CCT AAA TAC AAA TGC GAC  1782
Ala Tyr Arg Trp Lys Ser Thr Val Cys Met Val Met Pro Lys Tyr Lys Cys Asp   376

TTG TTT ACG TAC ATA GAT ATC ATG GGA CCA TTG CCA CTA AAT CAA ATA ATT ACG  1836
Leu Phe Thr Tyr Ile Asp Ile Met Gly Pro Leu Pro Leu Asn Gln Ile Ile Thr   394

ATA GAA CGG GGT TTG CTT GGA GCA TTG GCA TAT ATC CAC GAA AAG GGT ATA ATA  1890
Ile Glu Arg Gly Leu Leu Gly Ala Leu Ala Tyr Ile His Glu Lys Gly Ile Ile   412

CAT CGT GAT GTA AAA ACT GAA AAT ATA TTT TTG GAC AAA CCT GAA AAT GTA GTA  1944
His Arg Asp Val Lys Thr Glu Asn Ile Phe Leu Asp Lys Pro Glu Asn Val Val   430

TTG GGG GAC TTT GGG GCA GCA TGT AAA TTA GAT GAA CAT ACA GAT AAA CCC AAA  1998
Leu Gly Asp Phe Gly Ala Ala Cys Lys Leu Asp Glu His Thr Asp Lys Pro Lys   448

TGT TAT GGA TGG AGT GGA ACT CTG GAA ACC AAT TCG CCT GAA CTG CTT GCA CTT  2052
Cys Tyr Gly Trp Ser Gly Thr Leu Glu Thr Asn Ser Pro Glu Leu Leu Ala Leu   466

GAT CCA TAC TGT ACA AAA ACT GAT ATA TGG AGT GCA GGA TTA GTT CTG TTT GAG  2106
Asp Pro Tyr Cys Thr Lys Thr Asp Ile Trp Ser Ala Gly Leu Val Leu Phe Glu   484

ATG TCA GTA AAA AAT ATA ACC TTT TTT GGC AAA CAA GTA AAC GGC TCA GGT TCT  2160
Met Ser Val Lys Asn Ile Thr Phe Phe Gly Lys Gln Val Asn Gly Ser Gly Ser   502

CAG CTG AGA TCC ATA ATT AGA TGC CTG CAA GTC CAT CCG TTG GAA TTT CCA CAG  2214
Gln Leu Arg Ser Ile Ile Arg Cys Leu Gln Val His Pro Leu Glu Phe Pro Gln   520

AAC AAT TCT ACA AAC TTA TGC AAA CAC TTC AAG CAG TAC GCG ATT CAG TTA CGA  2268
Asn Asn Ser Thr Asn Leu Cys Lys His Phe Lys Gln Tyr Ala Ile Gln Leu Arg   538

CAT CCA TAT GCA ATC CCT CAG ATT ATA CGA AAG AGT GGT ATG ACG ATG GAT CTT  2322
His Pro Tyr Ala Ile Pro Gln Ile Ile Arg Lys Ser Gly Met Thr Met Asp Leu   556

GAA TAT GCT ATT GCA AAA ATG CTC ACA TTC GAT CAG GAG TTT AGA CCA TCT GCC  2376
Glu Tyr Ala Ile Ala Lys Met Leu Thr Phe Asp Gln Glu Phe Arg Pro Ser Ala   574

CAA GAT ATT TTA ATG TTG CCT CTT TTT ACT AAA GAA CCC GCT GAC GCA TTA TAC  2430
Gln Asp Ile Leu Met Leu Pro Leu Phe Thr Lys Glu Pro Ala Asp Ala Leu Tyr   592

ACG ATA ACT GCC GCT CAT ATG  TAAACACCCGTCAAAAATAACTTCAATGATTCATTTTATAATA 2494
Thr Ile Thr Ala Ala His Met                                                599

TATACTACGCGTTACCTGCAATAATGACAACATTCGAAGTCTTTGAAGATTCGCAGACCTTTTTTGCGAATG 2566
                                                                    Met  600

GCA CCT TCG GGA CCT ACG CCA TAT TCC CAC AGA CCG CAA ATA AAG CAT TAT GGA  2620
Ala Pro Ser Gly Pro Thr Pro Tyr Ser His Arg Pro Gln Ile Lys His Tyr Gly   618

ACA TTT TTG GAT TGC ATG AGA TAT ACT CTA AAC GAT GAG AGT AAG GTA GAT GAT  2674
Thr Phe Leu Asp Cys Met Arg Tyr Thr Leu Asn Asp Glu Ser Lys Val Asp Asp   636
```

```
AGA TGT TCA GAC ATA CAT AAC TCC TTA GCA CAA TCC AAT GTT ACT TCA AGC ATG   2728
Arg Cys Ser Asp Ile His Asn Ser Leu Ala Gln Ser Asn Val Thr Ser Ser Met    654

TCT GTA ATG AAC GAT TCG GAA GAA TAT CCA TTA ATA AAT GGA CCT TCG ATG CAG   2782
Ser Val Met Asn Asp Ser Glu Glu Tyr Pro Leu Ile Asn Gly Pro Ser Met Gln    672

GCA GAG GAC CCT AAA AGT GTT TTT TAT AAA GTT CGT AAG CCT GAC CGA AGT CGT   2836
Ala Glu Asp Pro Lys Ser Val Phe Tyr Lys Val Arg Lys Pro Asp Arg Ser Arg    690

GAT TTT TCA TGG CAA AAT CTG AAC TCC CAT GGC AAT AGT GGT CTA CGT CGT GAA   2890
Asp Phe Ser Trp Gln Asn Leu Asn Ser His Gly Asn Ser Gly Leu Arg Arg Glu    708

AAA TAT ATA CGT TCC TCT AAG AGG CGA TGG AAG AAT CCC GAG ATA TTT AAG GTA   2944
Lys Tyr Ile Arg Ser Ser Lys Arg Arg Trp Lys Asn Pro Glu Ile Phe Lys Val    726

TCT TTG AAA TGT GAA TCA ATT GGC GCT GGT AAC GGA ATA AAA ATT TCA TTC TCA   2998
Ser Leu Lys Cys Glu Ser Ile Gly Ala Gly Asn Gly Ile Lys Ile Ser Phe Ser    744

TTT TTC  TAACATTATAATATATCAGATCGTTTCTTATATACTTATTTTCATCGTCGGGATATGACTAAC 3067
Phe Phe                                                                    746

GTATACTAAGTTACAAGAAACAACTGCTTAACGTCGAACATAACGGAAATAAAAATATATATAGCGTCTCCT 3139

ATAANTGTTATATTGGCACCTTTTAGAGCTTCGGTATGAATAGATACAGATATG AAA GTA TTT TTT   3205
                                                       Met Lys Val Phe Phe    751

TTT AGA TAT ATC TCA TCC ACG AGA ATG ATT CTT ATA ATC TGT CTA CTT TTG GGA   3259
Phe Arg Tyr Ile Ser Ser Thr Arg Met Ile Leu Ile Ile Cys Leu Leu Leu Gly    769

ATT GGG GAC ATG TCC GCA ATG GGA CTT AAG AAA GAC AAT TCT CCG ATC ATT CCC   3313
Ile Gly Asp Met Ser Ala Met Gly Leu Lys Lys Asp Asn Ser Pro Ile Ile Pro    787

ACA TTA CAT CCG AAA GGT AAT GAA AAC CTC CGG GCT ACT CTC AAT GAA TAC AAA   3367
Thr Leu His Pro Lys Gly Asn Glu Asn Leu Arg Ala Thr Leu Asn Glu Tyr Lys    805

ATC CCG TCT CCA CTG TTT GAT ACA CTT GAC AAT TCA TAT GAG ACA AAA CAC GTA   3421
Ile Pro Ser Pro Leu Phe Asp Thr Leu Asp Asn Ser Tyr Glu Thr Lys His Val    823

ATA TAT ACG GAT AAT TGC AGT TTT GCT GTT TTG AAT CCA TTT GGC GAT CCG AAA   3475
Ile Tyr Thr Asp Asn Cys Ser Phe Ala Val Leu Asn Pro Phe Gly Asp Pro Lys    841

TAT ACG CTT CTC AGT TTA CTG TTG ATG GGA CGA CGC AAA TAT GAT GCT CTA GTC   3529
Tyr Thr Leu Leu Ser Leu Leu Leu Met Gly Arg Arg Lys Tyr Asp Ala Leu Val    859

GCA TGG TTT GTC TTG GGC AGA GCA TGT GGG AGA CCA ATT TAT TTA CGT GAA TAT   3583
Ala Trp Phe Val Leu Gly Arg Ala Cys Gly Arg Pro Ile Tyr Leu Arg Glu Tyr    877

GCC AAC TGC TCT ACT AAT GAA CCA TTT GGA ACT TGT AAA TTA AAG TCC CTA GGA   3637
Ala Asn Cys Ser Thr Asn Glu Pro Phe Gly Thr Cys Lys Leu Lys Ser Leu Gly    895

TGG TGG GAT AGA AGA TAT GCA ATG ACG AGT TAT ATC GAT CGA GAT GAA TTG AAA   3691
Trp Trp Asp Arg Arg Tyr Ala Met Thr Ser Tyr Ile Asp Arg Asp Glu Leu Lys    913

TTG ATT ATT GCA GCA CCC AGT CGT GAG CTA AGT GGA TTA TAT ACG CGT TTA ATA   3745
Leu Ile Ile Ala Ala Pro Ser Arg Glu Leu Ser Gly Leu Tyr Thr Arg Leu Ile    931

ATA ATT AAT GGA GAA CCC ATT TCG AGT GAC ATA TTA CTG ACT GTT AAA GAA ACA   3799
Ile Ile Asn Gly Glu Pro Ile Ser Ser Asp Ile Leu Leu Thr Val Lys Glu Thr    949

TGT AGT TTT TCG AGA CGG GGG ATA AAG GAT AAC AAA CTA TGC AAA CCG TTC AGT   3853
Cys Ser Phe Ser Arg Arg Gly Ile Lys Asp Asn Lys Leu Cys Lys Pro Phe Ser    967

TTT TTT GTC AAT GGT ACA ACA CGG CTG TTA GAC ATG GTG GGA ACA GGA ACC CCG   3907
Phe Phe Val Asn Gly Thr Thr Arg Leu Leu Asp Met Val Gly Thr Gly Thr Pro    985

AGA GCT CAT GAA GAA AAT GTG AAG CAG TGG CTT GAA CGA ATT GGT GGT AAA CAT   3961
```

```
Arg Ala His Glu Glu Asn Val Lys Gln Trp Leu Glu Arg Ile Gly Gly Lys His   1003

CTA CCA ATC GTC GTC GAA ACA TCT ATG CAA CAA GTC TCA AAT TTG CCG AGA AGT   4015
Leu Pro Ile Val Val Glu Thr Ser Met Gln Gln Val Ser Asn Leu Pro Arg Ser   1021

TTT AGA GAT TCA TAT TTC AAA TCA CCT GAC GAC GAT AAA TAT GAT GAC GTC AAA   4069
Phe Arg Asp Ser Tyr Phe Lys Ser Pro Asp Asp Asp Lys Tyr Asp Asp Val Lys   1039

ATG ACA TCG GCC ACT ACT AAT AAC ATT ACC ACC TCC GTG GAT GGT TAC ACT GGA   4123
Met Thr Ser Ala Thr Thr Asn Asn Ile Thr Thr Ser Val Asp Gly Tyr Thr Gly   1057

CTC ACT AAT CGG CCC GAG GAC TTT GAG AAA GCA CCA TAC ATA ACT AAA CGA CCG   4177
Leu Thr Asn Arg Pro Glu Asp Phe Glu Lys Ala Pro Tyr Ile Thr Lys Arg Pro   1075

ATA ATC TCT GTC GAG GAG GCA TCC AGT CAA TCA CCT AAA ATA TCA ACA GAA AAA   4231
Ile Ile Ser Val Glu Glu Ala Ser Ser Gln Ser Pro Lys Ile Ser Thr Glu Lys   1093

AAA TCC CGA ACG CAA ATA ATA ATT TCA CTA GTT GTT CTA TGC GTC ATG TTT TGT   4285
Lys Ser Arg Thr Gln Ile Ile Ile Ser Leu Val Val Leu Cys Val Met Phe Cys   1111

TTC ATT GTA ATC GGG TCT GGT ATA TGG ATC CTT CGC AAA CAC CGC AAA ACG GTG   4339
Phe Ile Val Ile Gly Ser Gly Ile Trp Ile Leu Arg Lys His Arg Lys Thr Val   1129

ATG TAT GAT AGA CGT CGT CCA TCA AGA CGG GCA TAT TCC CGC CTA  TAACACGTGTT 4395
Met Tyr Asp Arg Arg Arg Pro Ser Arg Arg Ala Tyr Ser Arg Leu               1144

TGGTATGGGCGTGTCGCTATAGTGCATAAGAAGTTGACTACATTGCATCAATGACATTATATAGCTTCTTTG 4467

GTCAGATAGACGGCGTGTGTGATTGCGATG TAT CTA CTA CAA TTA TTA TTT TGG ATC CGC   4527
                               Met Tyr Leu Leu Gln Leu Leu Phe Trp Ile Arg   1155

CTC TTT CGA GGC ATC TGG TCT ATA GTT TAT ACT GGA ACA TCT GTT ACG TTA TCA   4581
Leu Phe Arg Gly Ile Trp Ser Ile Val Tyr Thr Gly Thr Ser Val Thr Leu Ser   1173

ACG GAC CAA TCT GCT CTT GTT GCG TTC TGC GGA TTA GAT AAA ATG GTG AAT GTA   4635
Thr Asp Gln Ser Ala Leu Val Ala Phe Cys Gly Leu Asp Lys Met Val Asn Val   1191

CGC GGC CAA CTT TTA TTC CTG GGC GAC CAG ACT CGG ACC AGT TCT TAT ACA GGA   4689
Arg Gly Gln Leu Leu Phe Leu Gly Asp Gln Thr Arg Thr Ser Ser Tyr Thr Gly   1209

ACG ACG GAA ATC TTG AAA TGG GAT GAA GAA TAT AAA TGC TAT TCC GTT CTA CAT   4743
Thr Thr Glu Ile Leu Lys Trp Asp Glu Glu Tyr Lys Cys Tyr Ser Val Leu His   1227

GCG ACA TCA TAT ATG GAT TGT CCT GCT ATA GAC GCC ACG GTA TTC AGA GGC TGT   4797
Ala Thr Ser Tyr Met Asp Cys Pro Ala Ile Asp Ala Thr Val Phe Arg Gly Cys   1245

AGA GAC GCT GTG GTA TAT GCT CAA CCT CAT GAT AGA GTA CAA CCT TTT CCC GAA   4851
Arg Asp Ala Val Val Tyr Ala Gln Pro His Asp Arg Val Gln Pro Phe Pro Glu   1263

AAG GGA ACA TTG TTG AGA ATT GTC GAA CCC AGA GTA TCA GAT ACA GGC AGC TAT   4905
Lys Gly Thr Leu Leu Arg Ile Val Glu Pro Arg Val Ser Asp Thr Gly Ser Tyr   1281

TAC ATA CGT GTA GCT CTC GCT GGA AGA AAT ATG AGC GAT ATA TTT AGA ATG GCT   4959
Tyr Ile Arg Val Ala Leu Ala Gly Arg Asn Met Ser Asp Ile Phe Arg Met Ala   1299

GTT ATT ATA AGG AGT AGC AAA TCT TGG GCN TGT AAT CAC TCT GCT AGT TCA TTT   5013
Val Ile Ile Arg Ser Ser Lys Ser Trp  ?? Cys Asn His Ser Ala Ser Ser Phe   1317

CAG GCC CAT AAA TGT ATT CGC TAT GTC GAC CGT ATG GCC TTT GAA AAT TAT CTG   5067
Gln Ala His Lys Cys Ile Arg Tyr Val Asp Arg Met Ala Phe Glu Asn Tyr Leu   1335

ATT GGA CAT GTA GGC AAT TTG CTG GAC AGT GAC TCG GAA TTG CAT GCA ATT TAT   5121
Ile Gly His Val Gly Asn Leu Leu Asp Ser Asp Ser Glu Leu His Ala Ile Tyr   1353

AAT ATT ACT CCC CAA TCC ATT TCC ACA GAT ATT AAT ATT ATA ACG ACT CCA TTT   5175
Asn Ile Thr Pro Gln Ser Ile Ser Thr Asp Ile Asn Ile Ile Thr Thr Pro Phe   1371
```

```
TAC GAT AAT TCG GGA ACA ATT TAT TCA CCT ACG GTT TTT AAT TTG TTT AAT AAC  5229
Tyr Asp Asn Ser Gly Thr Ile Tyr Ser Pro Thr Val Phe Asn Leu Phe Asn Asn  1389

AAT TCC CAT GTC GAT GCA ATG AAT  TC                                      5255
Asn Ser His Val Asp Ala Met Asn                                          1397
```

SEQ ID n° 1 B

Longueur de la séquence : 1 188 paires de bases

Type de molécule séquencée : ADN génomique

Origine de la molécule : virus de la maladie de Marek souche RB1 B

Source expérimentale : plasmide pMDV 05

Caractéristique :

de 1 à 324 paires de bases : région non codante

de 325 à 1 135 paires de bases : gène US1 : le gène est transcrit de droite à gauche
la séquence indiquée est complémentaire à la séquence SEQ ID n° 1

```
                                                  Met Gly Val Ser
GAGATCAGACCGGCCCAGTTATTAACAATAAAAAGATTATTGGTGGAGTGAAG ATG GGT GTG TCC  1189

Met Ile Thr Ile Val Thr Leu Leu Asp Glu Cys Asp Arg Leu Pro Gly Arg Ser
ATG ATA ACT ATA GTC ACA CTT CTA GAT GAA TGC GAT CGA TTG CCA GGA AGA TCT 1113

Arg Asp Ala Ala Ser Thr Leu Trp Ile Phe Leu Ile Lys Gln Cys Met Glu Gln
AGA GAT GCT GCA TCT ACT TTA TGG ATA TTC CTT ATA AAG CAA TGT ATG GAA CAA 1069

Ile Gln Asp Asp Val Gly Val Pro Ile Ile Val Arg Ala Ala Asp Leu Phe Arg
ATA CAG GAT GAT GTG GGT GTG CCC ATA ATC GTC AGA GCT GCA GAC CTA TTC CGT 1015

Phe Ala Lys Pro Met Leu Ile Leu Pro Arg Gln His Arg Pro Ile Val Arg Thr
TTT GCC AAA CCC ATG TTA ATT CTT CCT CGG CAA CAT CGA CCG ATA GTA AGG ACA  961

Lys Pro Pro Asp Gly Thr Gly Val Arg Gly Thr Gly Leu Ala Gly Thr Arg Asp
AAG CCA CCA GAT GGA ACT GGA GTT CGT GGT ACC GGA TTG GCC GGA ACT AGG GAT  907

Ser Phe Ile Val Arg Leu Phe Glu Asp Val Ala Gly Cys Ser Thr Glu Trp Gln
TCG TTT ATA GTG CGG CTA TTT GAA GAT GTT GCA GGA TGT TCC ACA GAA TGG CAG  853

Asp Val Leu Ser Gly Tyr Leu Met Leu Glu Ser Glu Val Ser Gly Asn Ala Pro
GAT GTT CTA TCT GGA TAT TTG ATG TTG GAA TCT GAA GTT TCT GGT AAT GCT CCA  799

His Ser Leu Trp Ile Val Gly Ala Ala Asp Ile Cys Arg Ile Ala Leu Glu Cys
CAT AGC TTG TGG ATA GTT GGG GCG GCA GAT ATA TGT CGC ATT GCG CTC GAA TGT  745

Ile Pro Leu Pro Lys Arg Leu Leu Ala Ile Lys Val Ser Gly Thr Trp Ser Gly
ATT CCT TTG CCA AAA AGG TTA CTT GCA ATC AAA GTG TCT GGG ACC TGG TCC GGT  691

Met Pro Trp Ala Ile Pro Asp Asn Ile Gln Thr Leu Leu Thr Ser Thr Trp Glu
ATG CCG TGG GCC ATT CCC GAC AAT ATT CAA ACT CTC TTG ACA TCT ACA TGG GAA  637

Pro Lys Phe Asp Thr Pro Glu Asp Arg Ala His Phe Cys Asp Ser Asp Met Val
CCG AAG TTC GAC ACC CCA GAA GAT AGA GCG CAT TTT TGC GAC AGT GAT ATG GTA  583

Cys Val Tyr Lys Ile Leu Gly Ser Pro Pro Asn Pro Leu Lys Pro Pro Glu Ile
TGT GTA TAC AAA ATC CTC GGG TCC CCA CCC AAT CCT CTA AAA CCT CCG GAA ATC  529

Glu Pro Pro Gln Met Ser Ser Thr Pro Gly Arg Leu Phe Cys Cys Gly Lys Cys
GAA CCA CCT CAA ATG AGT AGT ACA CCC GGC AGA TTA TTC TGT TGT GGA AAA TGT  475

Cys Lys Lys Glu Asp Arg Asp Ala Ile Ala Ile Pro Val Arg Tyr Thr Ala Thr
TGC AAG AAA GAA GAT AGA GAT GCG ATT GCA ATT CCG GTT CGT TAC ACT GCG ACA  367

Gly Lys Ser Arg Ile Gln Lys Lys Cys Arg Ala Gly Ser His
GGA AAG TCA CGA ATA CAG AAA AAA TGT AGA GCC GGT AGT CAT  TAGCTGTTATTCGAC 310

AGACCTACTTGCTACCAATTAGATATAATTACATGATGGGGCGTATACACATTACGATTAGGTGCATCGCTA 238

CAACCGTCGCTATAGTGTCACGTATAATTTGTATATTACTGCAATAACAAACCCTTCTAGATCACTTATGTA 166

TCCAGGCTATCTTCCATATACTTCTAACATCAGGAGAGATTCAACAATCGAGCGCATTTGAAAGACAACGAT  94

GAGCAGAGTCAATGCTACAATGTTCGATGATATGGATATACCAAGAGGACGATTTGGTAAGCCACCGAGAAA  22

GATTACTAATGTAAATTTTTG                                                      1
```

SEQ ID n° 2

Type de séquence : oligonucléotide

Longueur de la séquence

Type de molécule : ADN

5′ GGA CTC GAA CCA TGG AGT CTA TTA CC 3′

NCO 1

SEQ ID n° 3

Type de séquence : oligonucléotide

Longueur de la séquence

Type de molécule : ADN

5′ GAC GGG TGT CGA CAT ATG AG 3′

SalI

SEQ ID n° 4

Type de séquence : oligonucléotide

Longueur de la séquence

Type de molécule : ADN

5′ CTG GAG CCC ATG GTG CAC CTT TG 3′

NCO1

SEQ ID n° 5

Type de séquence : oligonucléotide

Longueur de la séquence

Type de molécule : ADN

5′ CAA ATT GCT ATT GTC GAC ACC TCC GCC TCT C 3′

SalI

## Revendications

**1)** Séquence nucléotidique, et ses variantes, du domaine court unique (Us) du virus de la maladie de Marek, caractérisée en ce qu'elle correspond au gène US3, non essentiel à la replication, et homologue du gène de la protéine kinase du virus herpès simplex.

**2)** Séquence nucléotidique US3 du virus MDV, et ses variantes, selon la revendication 1 apparaissant sur la séquence ID n° 1.

**3)** Séquence nucléotidique ID n° 1 et gènes et leurs variantes appartenant à cette séquence.

**4)** Virus recombinant choisi parmi les virus herpès, comprenant au moins un gène hétérologue inséré dans la région Us du génome dudit virus, correspondant au gène de la protéine kinase.

**5)** Virus recombinant selon la revendication 4, caractérisé en ce que le gène est un gène codant pour un immunogène viral, bactérien ou parasitaire.

**6)** Virus recombinant de la maladie de Marek, selon la revendication 5, comportant au moins un gène hétérologue, caractérisé en ce que ce gène est inséré dans la région de son génome correspondant au gène US3, de manière à pouvoir s'exprimer.

**7)** Virus recombinant selon la revendication 6, caractérisé en ce que le gène hétérologue inséré code pour un pathogène choisi dans le groupe constitué par la bronchite infectieuse aviaire, la maladie de Newcastle, la maladie de Gumboro, la peste aviaire, l'anémie aviaire, le syndrome chute de ponte, la variole aviaire, la laryngotrachéite infectieuse, la colibacillose, la pasteurelose, la coccidiose, l'hémophilose.

**8)** Virus recombinant selon l'une des revendications 6 et 7, caractérisé en ce qu'il s'agit du virus MDV.

**9)** Virus recombinant selon l'une des revendications 6 et 7, caractérisé en ce qu'il s'agit du virus HVT.

**10)** Virus recombinant selon la revendication 9, caractérisé en ce que le gène inséré code pour un immunogène d'un virus de la maladie de Marek de sérotype 1 ou 2.

**11)** Virus recombinant selon l'une quelconque des revendications 4 à 10, caractérisé en ce que le gène est inséré pour être exprimé sous le contrôle des séquences régulatrices de transcription du gène de la protéine kinase.

**12)** Virus recombinant selon l'une des revendications 4 à 11, caractérisé en ce que le gène hétérologue inséré est susceptible d'être exprimé sous la dépendance de séquences promotrices du virus considéré, ou d'autres virus herpès.

**13)** Virus recombinant selon l'une quelconque des revendications 4 à 12, caractérisé en ce que les codons. d'initiation et de terminaison du gène inséré sont substitués à ceux du gène US3.

**14)** Vaccin caractérisé en ce qu'il comprend un virus recombinant selon l'une quelconque des revendications 3 à 9.

**15)** Procédé de préparation d'un virus recombinant de la maladie de Marek, caractérisé en ce que l'on insère au moins un gène hétérologue dans la région de son génome correspondant au gène US3, de manière à pouvoir s'exprimer.

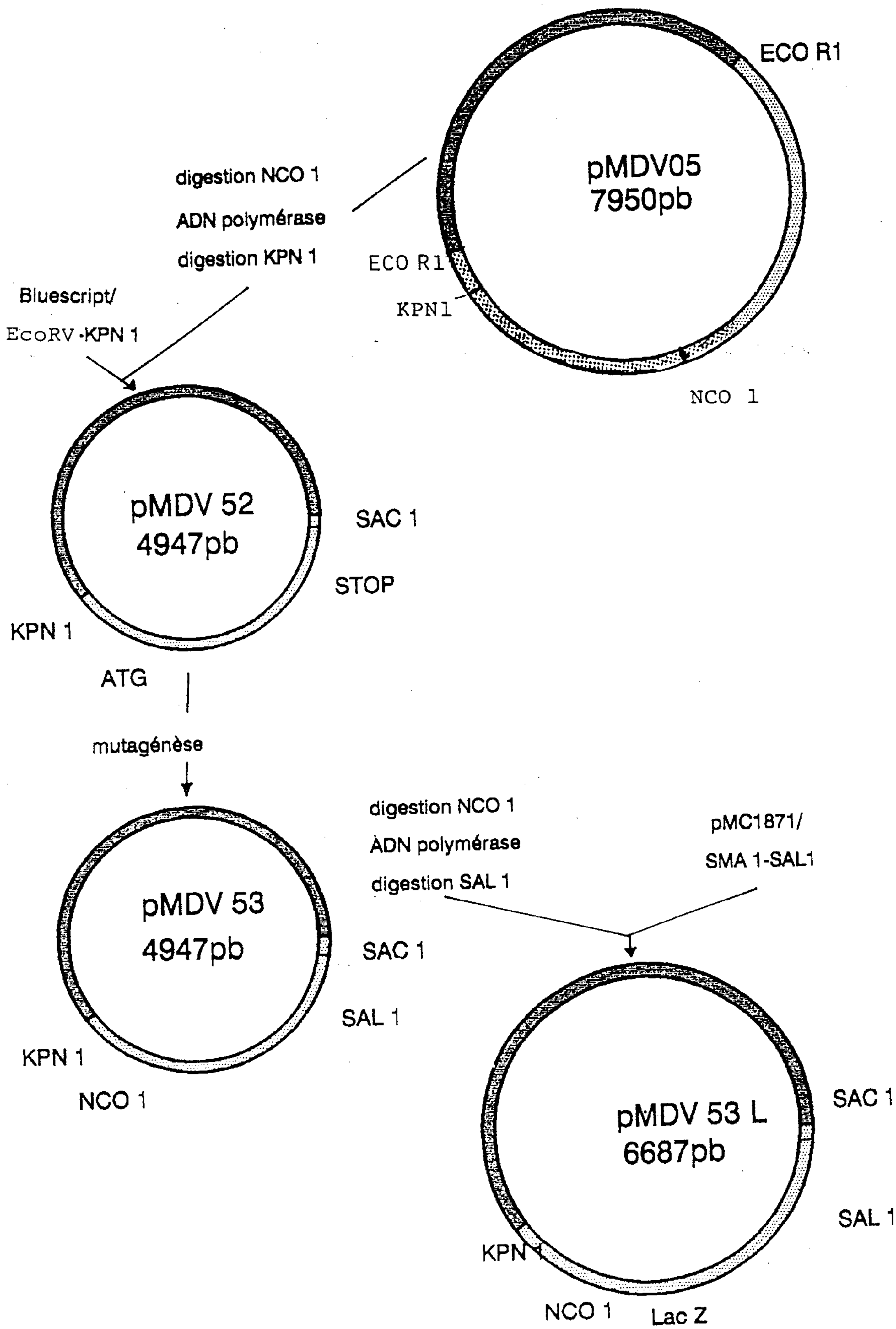
ECO R1
pMDV05
7950pb
digestion NCO 1
ADN polymérase
digestion KPN 1
ECO R1
KPN1
NCO 1
Bluescript/
EcoRV-KPN 1
pMDV 52
4947pb
SAC 1
STOP
KPN 1
ATG
mutagénèse
digestion NCO 1
ADN polymérase
digestion SAL 1
pMC1871/
SMA 1-SAL1
pMDV 53
4947pb
SAC 1
SAL 1
KPN 1
NCO 1
pMDV 53 L
6687pb
SAC 1
SAL 1
KPN 1
NCO 1
Lac Z

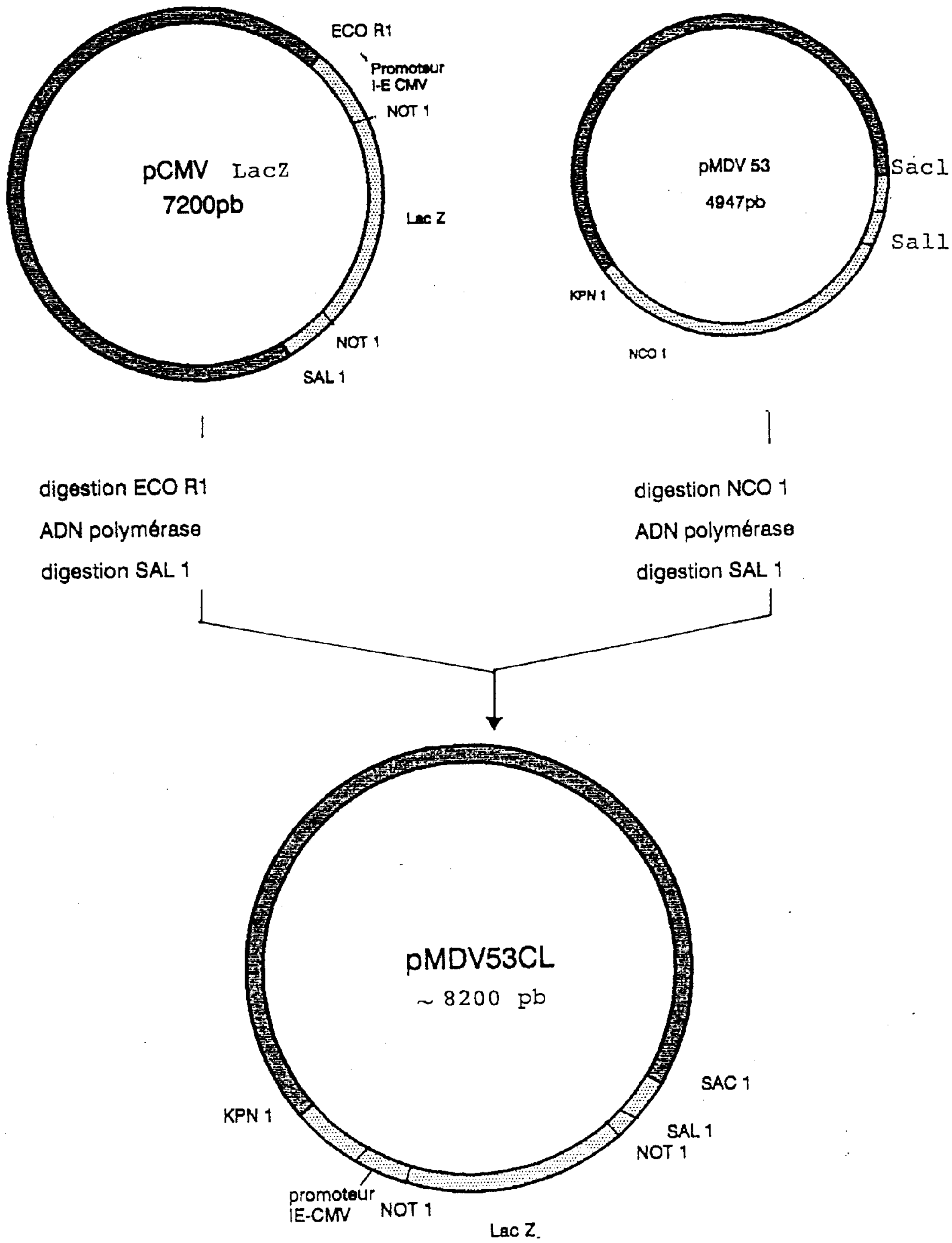

ECO R1
Promoteur
I-E CMV
NOT 1
pCMV LacZ
7200pb
Lac Z
NOT 1
SAL 1
pMDV 53
4947pb
SacI
SalI
KPN 1
NCO 1
digestion ECO R1
ADN polymérase
digestion SAL 1
digestion NCO 1
ADN polymérase
digestion SAL 1
pMDV53CL
~ 8200 pb
SAC 1
KPN 1
SAL 1
NOT 1
promoteur
IE-CMV
NOT 1
Lac Z.

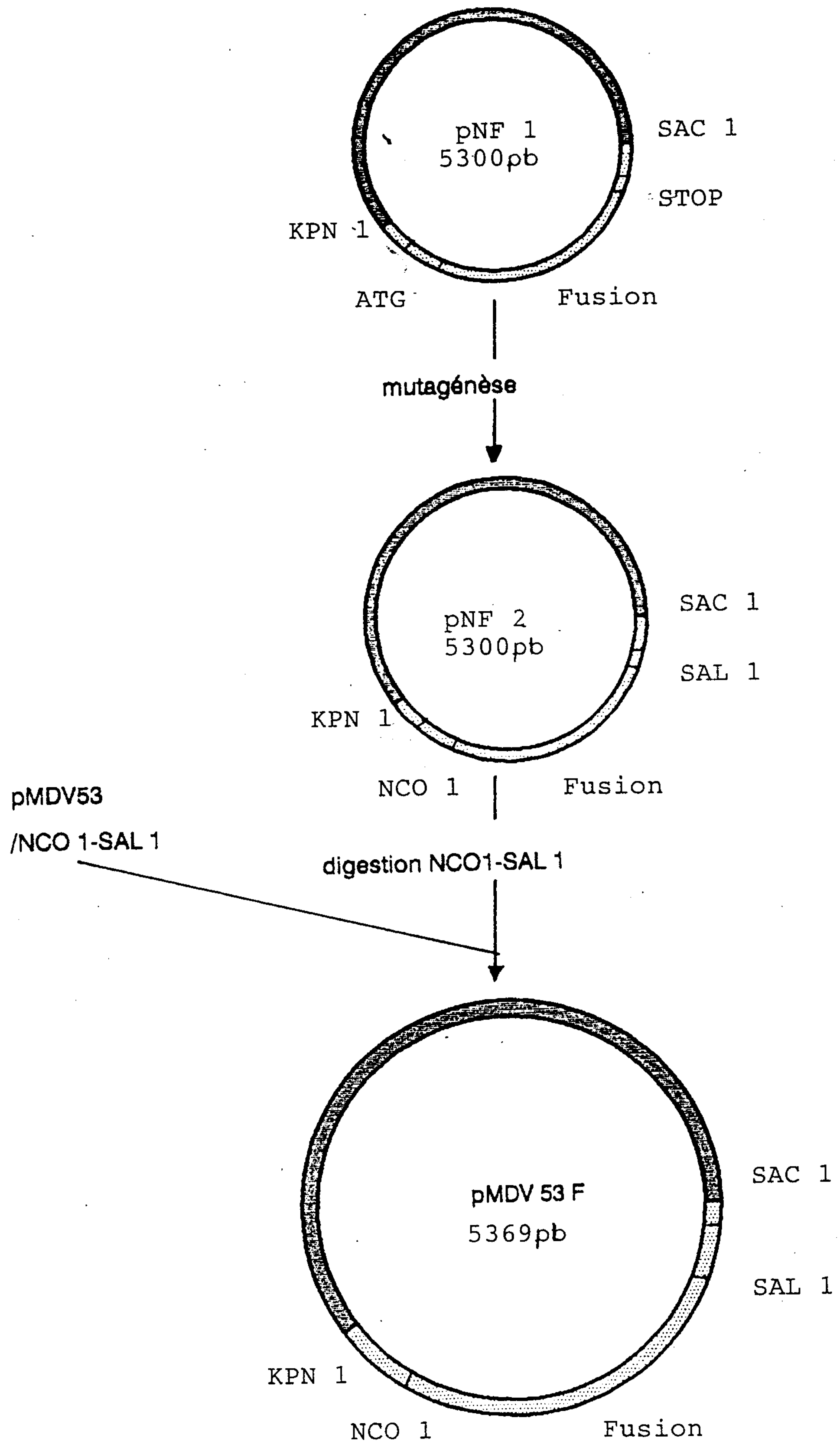
pNF 1
5300pb
SAC 1
STOP
KPN 1
ATG
Fusion
mutagénèse
pNF 2
5300pb
SAC 1
SAL 1
KPN 1
NCO 1
Fusion
pMDV53
/NCO 1-SAL 1
digestion NCO1-SAL 1
pMDV 53 F
5369pb
SAC 1
SAL 1
KPN 1
NCO 1
Fusion

Office européen des brevets

# RAPPORT DE RECHERCHE EUROPEENNE

Numero de la demande

EP 91 40 2336

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | JOURNAL OF VIROLOGY, vol. 61, no. 9, septembre 1987, pages 2896-2901; F.C. PURVES et al.: "Herpes simplex virus 1 protein kinase is encoded by open reading frame US3 which is not essential for virus growth in cell culture" * Le document en entier * --- | 4,5 | C 12 N 15/86<br>C 12 N 9/12<br>C 12 N 15/38<br>A 61 K 39/255<br>A 61 K 39/17 |
| Y | IDEM --- | 1,6-15 | |
| X,D | JOURNAL OF GENERAL VIROLOGY, vol. 69, 1988, pages 2033-2042; A.E. BUCKMASTER et al.: "Gene sequence and mapping data from Marek's disease virus and herpesvirus of turkeys: Implications for herpesvirus classification" * Le document en entier * --- | 1-3 | |
| Y | IDEM --- | 4-15 | **DOMAINES TECHNIQUES RECHERCHES (Int. Cl.5)** |
| Y | WO-A-9 002 802 (INSTITUTE FOR ANIMAL HEALTH LTD) * Le document en entier * --- | 1,4-15 | C 12 N<br>C 07 K |
| P,X | JOURNAL OF GENERAL VIROLOGY, vol. 72, partie 4, avril 1991, pages 939-947; L.J.N. ROSS et al.: "Properties and evolutionary relationships of the Marek's disease virus homologues of protein kinase, glycoprotein D and glycoprotein I of herpes simplex virus" * Le document en entier * --- -/- | 1-3 | |

Le présent rapport a été établi pour toutes les revendications

| Lieu de la recherche | Date d'achèvement de la recherche | Examinateur |
|---|---|---|
| LA HAYE | 06-01-1992 | CHAMBONNET F.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Page 2

Office européen des brevets

# RAPPORT DE RECHERCHE EUROPEENNE

Numero de la demande

EP 91 40 2336

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| P,X | JOURNAL OF GENERAL VIROLOGY, vol. 72, partie 4, avril 1991, pages 949-954; L.J.N. ROSS et al.: "DNA sequence and organization of genes in a 5.5 kbp EcoRI fragment mapping in the short unique segment of Marek's disease virus (strain RB1B)" * Le document en entier * | 1-3 | |
| Y | JOURNAL OF GENERAL VIROLOGY, vol. 71, no. 8, août 1990, pages 1747-1755; M. VAN ZIJL et al.: "Identification of two genes in the unique short region of pseudorabies virus; comparison with herpes simplex virus and varicella-zoster virus" * Le document en entier * | 4 | |
| Y | JOURNAL OF GENERAL VIROLOGY, vol. 68, 1987, pages 2699-2704; M.C. FRAME et al.: "Identification of the herpes simplex virus protein kinase as the product of viral gene US3" * Le document en entier * | 4 | DOMAINES TECHNIQUES RECHERCHES (Int. Cl.5) |

Le présent rapport a été établi pour toutes les revendications

| Lieu de la recherche | Date d'achèvement de la recherche | Examinateur |
|---|---|---|
| LA HAYE | 06-01-1992 | CHAMBONNET F.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)